Europäisches Patentamt

European Patent Office

Office européen des brevets

(11) Publication number: **0 511 933 A2**

# EUROPEAN PATENT APPLICATION

(21) Application number: **92610027.2**

(22) Date of filing: **14.04.92**

(51) Int. Cl.5: **C12N 1/20**, C12N 9/24, D21C 3/00, D21H 17/00, //(C12N1/20,C12R1:01)

The applicant has filed a statement in accordance with Rule 28 (4) EPC (issue of a sample only to an expert). Accession number(s) of the deposit(s): DSM 6262.

(30) Priority: **18.04.91 DK 695/91**

(43) Date of publication of application: **04.11.92 Bulletin 92/45**

(84) Designated Contracting States: **PT**

(71) Applicant: **NOVO NORDISK A/S**
**Novo Allé**
**DK-2880 Bagsvaerd(DK)**

(72) Inventor: **Mathrani, Indra M.**
**Elmevaenget 5A**
**DK-2880 Bagsvaerd(DK)**
Inventor: **Ahring, Birgitte K.**
**Elmevaenget 5A**
**DK-2880 Bagsvaerd(DK)**
Inventor: **Anker, Lisbeth**
**Athensvej 16**
**DK-2300 Copenhagen S(DK)**

(54) Dictyoglomus microorganisms and their xylanolytic enzymes.

(57) This invention relates to novel microorganisms and to novel enzymes. More specifically, the invention relates to a new species of the genus *Dictyoglomus*, and to novel enzymes having xylanolytic activity obtainable from the genus *Dictyoglomus*. The invention also relates to the use of these novel xylanolytic enzymes in pulp and paper industry.

EP 0 511 933 A2

## TECHNICAL FIELD

This invention relates to novel microorganisms and to novel enzymes. More specifically, the invention relates to a new species of the genus *Dictyoglomus*, and to novel enzymes having xylanolytic activity obtainable from the genus *Dictyoglomus*. The invention also relates to the use of these novel xylanolytic enzymes in pulp and paper industry.

## BACKGROUND ART

Only three isolates of *Dictyoglomus* are reported [*Patel, B.K., Morgan, H.W., Wiegel, J., and Daniel, R.M.* (1987); Arch. Microbiol. **147** 21-24; *Saiki, T., Kobayashi, Y., Kawagoe, K., and Beppu, T.* (1985); Int. J. Syst. Bacteriol. **35** 253-259; and *Svetlichnii, V.A. and Svetlichnaya, T.P.* (1988); Mikrobiologiya (Engl. Translation) **57** 364-370 (*D. turgidus*)], and *D. thermophilum* (*Saiki et al.*, 1985) is the only valid member of its genus and the only published species which resembles the microorganisms of this invention. *D. thermophilum* is a thermophilic, strictly anaerobic, chemorganotrophic eubacterium that was isolated from a natural hot spring, whereas the microorganisms of this invention were isolated from a man-made environment well removed from any natural thermophilic site. *D. thermophilum* uses a wide range of carbohydrates for growth, unlike the organisms of this invention, which use only xylan. During growth, *D. thermophilum* produces significant quantities of acetate, lactate, and $CO_2$, and some $H_2$ and ethanol as fermentation products. The organisms of this invention make only acetate as a major fermentation product during growth and produce small quantities, 1 mM or less, of $H_2$ and $CO_2$. Additionally, in stationary phase cultures of the microorganisms of this invention produce some butyrate, a compound not previously reported as a fermentation product Dictyoglomus. Both organisms of this invention and *D. thermophilum* are rod-shaped and form spherical bodies, normally formed by only them and one other group of unrelated organisms. The reported guanine/cytosine content of the cellular deoxyribonucleic acid of *D. thermophilum*, a major genotypic characteristic of bacteria, is 29% (measured by thermal denaturation), significantly different from that of the organisms of this invention which have a guanine/cytosine content of 34% as measured by High Performance Liquid Chromatography. *D. thermophilum* has thermostable amylases which have been cloned into *E. coli*. The organisms of this invention do not grow with amylose as substrate and amylase activity has not been detected. One report mentioned the detection of a single xylanase in a *Dictyoglomus* like organism, but it was unclear whether this was demonstrated in *D. thermophilum*.

Given the restricted substrate utilization of the organisms of this invention, its previously undescribed fermentation products, and its different guanine/cytosine ratio of the cellular deoxyribonucleic acid, the organisms of this invention represent at least a new species of the genus *Dictyoglomus*. In addition, the xylanases we have identified from the organisms of this invention are unlike any enzymes described for any Dictyoglomus reported in the literature. Particularly, they possess especially good characteristics which make them applicable for use in the production of paper pulp and paper.

## SUMMARY OF THE INVENTION

In its first aspect, the invention relates to previously undescribed microorganisms belonging to a new species of the genus *Dictyoglomus*.

In another aspect, the invention relates to novel enzymes having xylanase activity and having a broad pH optimum in the range of from 5.0 to 9.0 (after 20 minutes at 60°C), temperature optimum in the range of from 60 to 90°C (after 20 minutes at pH 6.0), and immunochemical properties identical or partially identical to those of a xylanase derived from Strain B1, DSM No. 6262.

In its third aspect, the invention provides a process for the preparation of an enzyme according to the invention, comprising cultivation of a xylanase producing strain of an organism of the invention in a suitable nutrient medium, containing carbon and nitrogen sources and inorganic salts, followed by recovery of the desired enzyme by methods known *per se*.

In its fourth aspect, the invention provides a process for treatment of lignocellulosic pulp, in which the lignocellulosic pulp is treated with an enzyme of the invention.

In its fifth aspect, the invention provides an agent for use in the treatment of lignocellulosic pulp, which agent contains an enzyme of the invention.

## BRIEF DESCRIPTION OF DRAWINGS

The present invention is further illustrated by reference to the accompanying drawings, in which:

Fig. 1 shows the pH curve for growth of an organism of this invention;

Fig. 2 shows the temperature curve for growth of an organism of this invention;

Fig. 3 shows the pH dependent activity (% rel.) of an enzyme preparation of the invention, obtained from a culture supernatant incubated at 68°C (■ Strain B1 xylanase preparation; ▲ *D. thermophilum*, DSM 3960, xylanase preparation);

Fig. 4 shows the temperature dependent activity (% rel.) of an enzyme preparation of the invention, obtained from cultivation of Strain B1 (■ Supernatant from culture incubated at 68°C; ● Supernatant from culture incubated at 78°C);

Fig. 5 shows the temperature dependent activity (% rel.) of an enzyme preparation of the invention, obtained from cultivation of the strain *D. thermophilum*, DSM 3960, (■ Supernatant from culture incubated at 68°C; □ Supernatant from culture incubated at 78°C);

Fig. 6A shows the activity remaining (% rel.) of an enzyme preparation of the invention, obtained from cultivation at 68°C of Strain B1 after heat-treatment at the temperature indicated (○ 70°C; ► 80°C; ● 90°C; and ■ 98°C);

Fig. 6B shows a half-logarithmic plot of half-lives. The arrow indicates the thermal activity number (89°C);

Fig. 7 shows the pH curve of the xylanolytic activity (% rel.) of an enzyme preparation of the invention;

Fig. 8 shows the temperature curve of the xylanolytic activity (% rel.) of an enzyme preparation of the invention;

Fig. 9 shows the residual activity curve of the xylanolytic activity (% rel.) of an enzyme preparation of the invention;

Fig. 10 shows the thermal stability of the xylanolytic activity (% rel.) of an enzyme preparation of the invention;

Fig. 11 shows an absorbance scan curve illustrating the lignin release to the filtrate after treatment with an enzyme preparation of the invention; and

Fig. 12 shows the monosaccharide composition of the acid hydrolyzed filtrates obtained after treatment with an enzyme preparation of the invention.

## DETAILED DISCLOSURE OF THE INVENTION

### The Microorganisms

In its first aspect, the invention relates to novel microorganisms belonging to the genus *Dictyoglomus*.

Microorganisms were collected at Kirkniemi, Finland. The sample was pulp-mass and water from a pulp-mass cooling tank of a pulp factory. Microorganisms were isolated from a 70°C, pH 8.3 enrichment culture with beech xylan as substrate.

A representative isolate of these novel microorganisms of the invention, designated Strain B1, has been deposited on 28 November 1990 at the DSM, Deutsche Sammlung von Mikroorganismen und Zellkulturen GmbH, Mascheroder Weg 1 B, D-3300 Braunsweig, Germany, for the purpose of patent procedures according to the Budapest Treaty, and is given the accession number DSM 6262.

The organisms have been characterized by *Indra M. Mathrani*, Ph.D., and *Birgitte K. Ahring*, Ph.D., and the characterization was confirmed by Dr. *Hans Hippe*, Director of the German Collection of Microorganisms and Cell Cultures.

The type culture of *Dictyoglomus thermophilum* has been deposited in 1985, and hence are publicly available from Deutsche Sammlung von Mirkoorganismen und Zellkulturen GmbH, Mascheroder Weg 1b, D-3300 Braunschweig, Germany, with the accession number DSM 3960.

### Colony And Cell Morphology Of The Organisms Of Invention

In anaerobic roll tubes, containing gel-solidified (Gelrite®) medium at pH 8.2 with beech tree xylan (4 g/l) as substrate, the surface colonies are tan and circular with an entire edge and a convex shape, subsurface colonies are lens shaped. The colonies have an iridescent, grainy interior, and microscopic examination of picked colonies shows that the long, thin cells (rod-shaped) of the culture lie side by side in large parallel ranks. These parallel ranks of cells may refract light and produce the iridescent colouring of the colonies.

Liquid cultures of the microorganisms in exponential phase cultures contain cells, 5 to 20 μm in length and 0.3 μm wide, with rounded ends. Cells occur singly, in pairs, and in bundles lying side by side. Cells in exponential phase do not stain Gram positive and do not bind safranine well, and thus are nearly colourless after Gram Stain preparation. Endospores are not observed and the organism is not motile. Cultures do not spontaneously form spheroplasts, and spheroplasts are not induced by lysozyme. However, cells are sensitive to lysis by lysozyme, as evidential by release of DNA or by microscopic observation. In late exponential phase cultures the cell bundles are often swollen, and intermediate forms up to a spherical, "ball of yarn" structures with cells lying on the surface of the sphere are observed. The spherical structures have a diameter of from 5 to 25 $\mu$m and careful examination of the ball structures at high magnification shows that the ball structure has a membrane or wall, and in older cultures cells can be seen peeling away from the ball structures and naked spheres of all sizes are present. In exponential phase cultures the ball structures are empty but in stationary phase cultures a small number of the larger spheres, particularly those from which cells have peeled off, contained small amounts of heterogenous material. The spherical structures are sensitive to lysis by sodium dodecyl sulfate, 3% wt/vol, but the cells are stable, in sodium dodecyl sulfate treated samples, cells are whole, do not appear lysed, though they are not as dark as normal when observed with phase contrast microscopy, are well dispersed, with no bundles or spheres visible.

## Scanning Electron Microscopy

Scanning electron micrographs prepared by cryogenic freezing fixation of late exponential phase cultures of the microorganism shows single cells, alone and in bundles, ball structures, and extracellular, slime-like material around the cells in the spherical structures and on the single cells. During preparation of the samples sublimation of water causes the collapse of the ball structures leaving flattened spheroids, the cells measure 0.24 $\mu$m in width after cryogenic preparation. The "ball of yarn" nature of the ball structure is easily visible, with cells wrapped around the sphere and lying in parallel, side by side on the surface.

## Transmission Electron Micrographs

Thin sections of late exponential phase cultures examined with transmission electron microscopy show that the microorganism has a thick, extracellular coat not visible with light microscopy and distinct from the cell wall that is visible outside the wall of the cell. No special interior features within the cells are visible. Thin sections through ball structures showed that they are empty and no membranous or wall-like structure lines of the interior of the ball are present after preparation, as observed with phase contrast microscopy.

## Substrate and Nutritional Requirements

Of all substrates tested, the microorganisms of the invention will only use xylans, beech tree or oat spelt, as carbon and energy sources. There is no growth or metabolism with xylose, xylobiose, arabinose, glucose, fructose, sucrose, galactose, mannose, maltose, rhamnose, lactose, starch, cellobiose, cellulose, peptone, and yeast extract. In addition a xylose syrup from Cerestar® (containing: Xylose, 48-55%; Arabinose, 9-13%; Rhamnose, 3-5%; Mannose, 0.5-1.5%; Galactose, 3-6%; and Glucose, 8-13%) is tested as substrate. All substrates are tested at 4 and 1 g xylan per litre. Major fermentation products from xylan are acetic acid (up to 25 mM), trace levels of hydrogen gas (up to 1 mM), and carbon dioxide. Lactate, formate, or ethanol are not detectable (detection limit 0.25 mM).

Growth is stimulated by the presence of both yeast extract and vitamins, however, neither are required for growth.

For comparison, in Table 1 some major characteristics of extant *Dictyoglomus* strains are presented.

## Optimum pH and Temperature for Growth

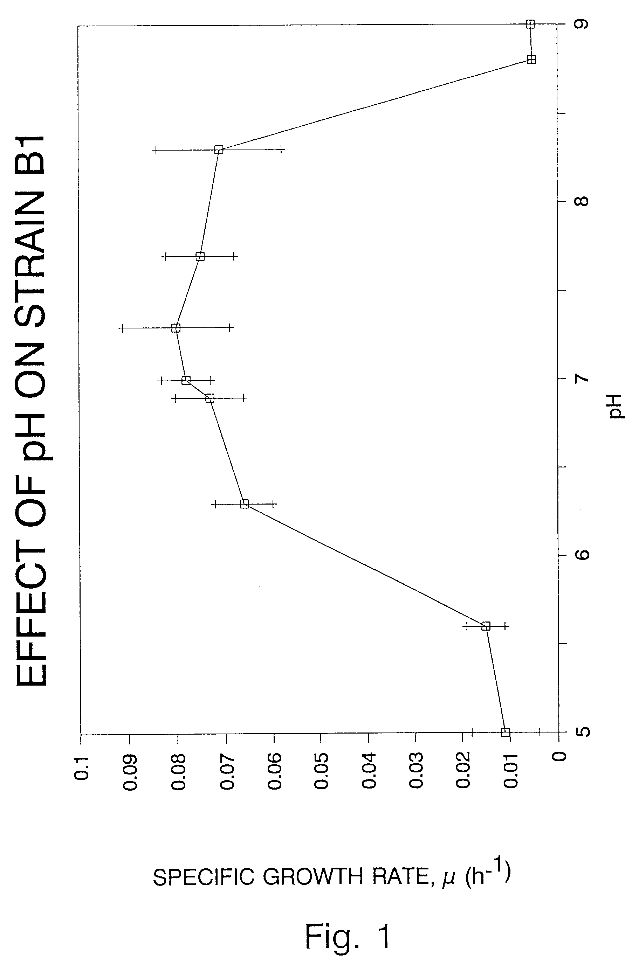

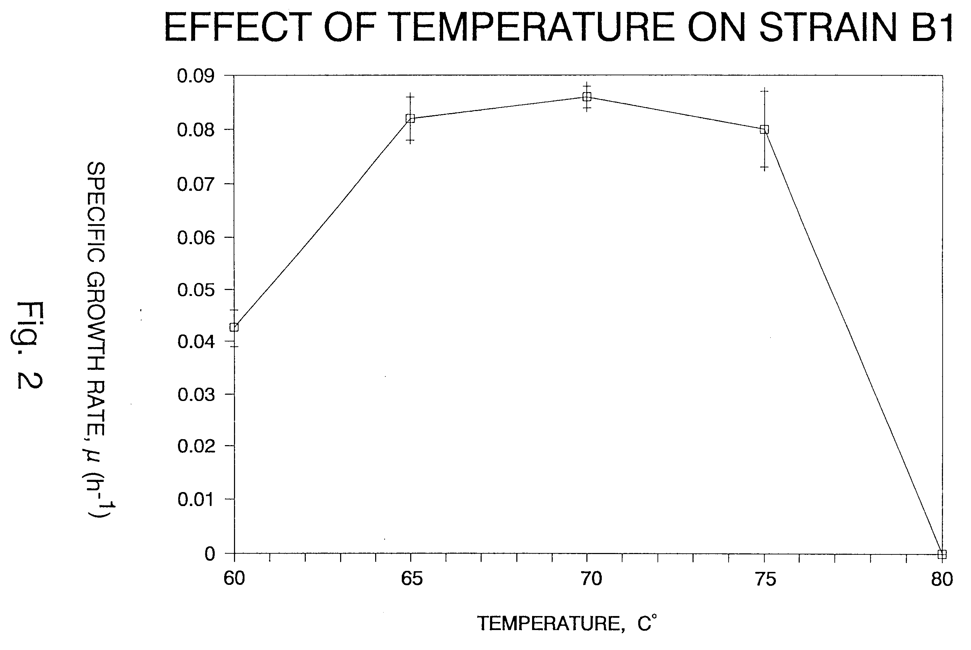

Microorganisms of this invention are isolated at a relatively high pH and this is reflected in its ability to grow at pH values well over 8.0 and near 9. Optimum growth occurs over a wide range, from pH near 6 to over 8 (Fig. 1), with slow growth at pH 5.0 and no growth at pH 9. The effect of temperature on the growth rate of the microorganisms is shown in Fig. 2. Growth occurs from below 60°C up to over 75°C, but not at 80°C. Best growth occurs from under 65 to over 75°C.

## Antibiotic Sensitivity

The antibiotic sensitivity of the microorganisms of the invention is similar to that of typical eubacteria. It is sensitive to chloramphenicol, kanamycin, penicillin, streptomycin, tetracycline, and vancomycin, all at 100 mg/l. However, the microorganisms are unaffected by ampicillin, chloramphenicol, and tetracycline at 10 mg/l.

GC-content Determination

The guanosine and cytosine content of the total cellular DNA of the organisms of the invention was determined by HPLC-analysis. In this way the average GC-content measured was 34 mol-%.

Table 1

Characteristics of Extant Dictyoglomus Strains

| Isolates | Substrates used | Fermentation products[a] (substrate) | G+C% | Source | Reference |
|---|---|---|---|---|---|
| B1 | xylans only | $Ac, H_2, CO_2$ (beech xylan) | 34 | pulp mill cooling tank | An organism of this invention |
| Dictyoglomus thermophilum | numerous carbohydrates[b] | $Ac, Lac, H_2, CO_2$ (starch) | 29 | hot-spring | Saiki et al. 1985 |
| RT46-B1 | numerous carbohydrates[b] | $Ac, EtOH, Lac, H_2, CO_2$ (glucose) | 29.5 | hot-spring | Patel et al. 1987 |
| Z-1310 | numerous carbohydrates[c] | $Ac, EtOH, Lac, H_2, CO_2$ (starch) | 32.5 | hot-spring | Svetlichnii and Svetlichnaya 1988 |

[a] Ac = acetate, EtOH = ethanol, Lac = lactate
[b] Xylan degradation reported (Patel et al., 1987)
[c] Does not use xylose, xylan degradation not tested>

The Enzyme Preparations

In its second aspect, the invention relates to novel enzyme preparations having xylanolytic activity.

The xylanase preparations of this invention are produceable by cultivation of a microorganism of the invention, preferably by cultivation of a strain of microorganisms essentially identical to the strain DSM No. 6262, or a mutant or a variant thereof. The xylanase preparations of this invention are also produceable by cultivation of the strain *D. thermophilum*, DSM 3960, or a mutant or a variant thereof. Most likely these orgamisms are able to produce a complex of xylanases, as indicated by the broad pH range and the pI values determined.

The xylanase preparations of this invention can also be obtained by recombinant DNA-technology.

The xylanase preparations of the invention can be described by the following characteristics.

## Physical-chemical Properties

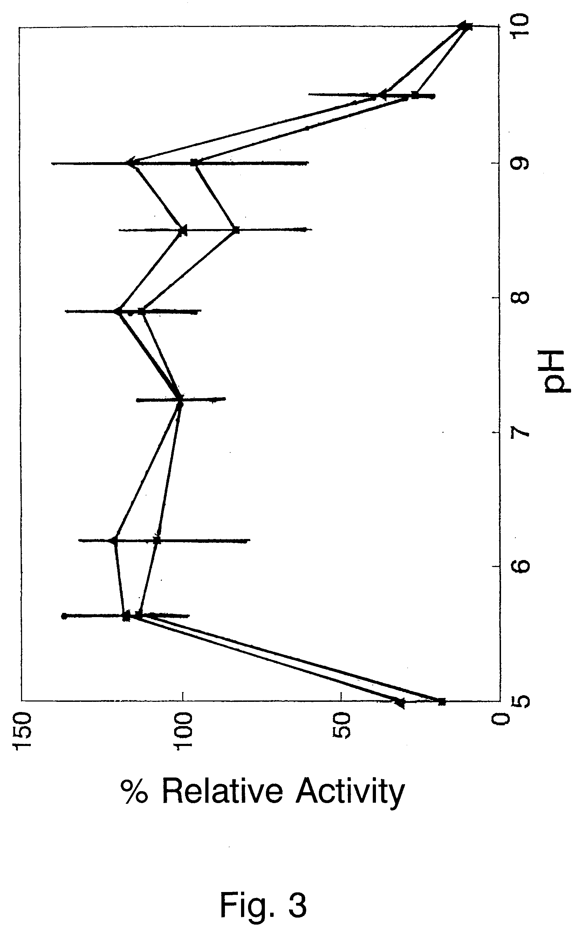

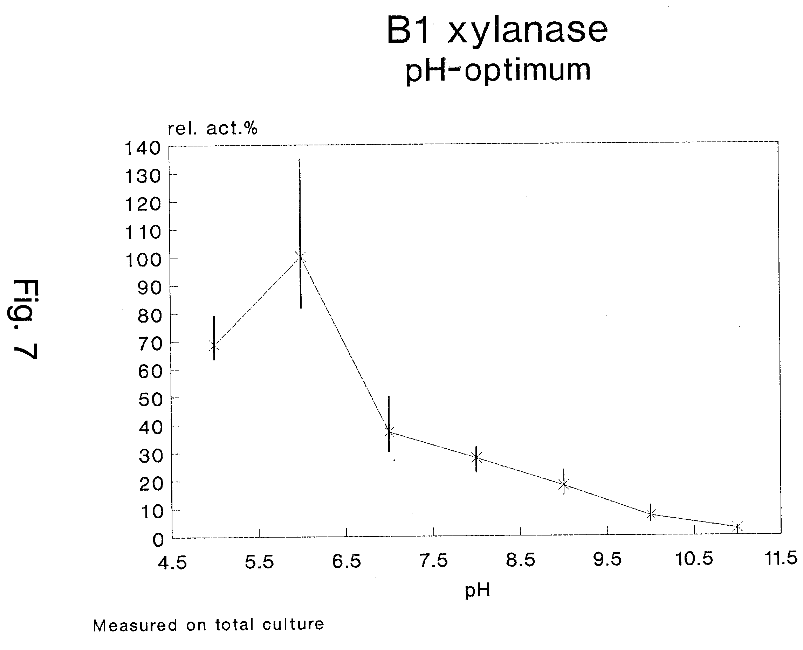

The influence of pH on the xylanolytic activity was determined according to Examples 2-3, using the method for xylanase activity analysis described in this specification (20 minutes of incubation; 60°C). The result of the determination is presented in Figs. 3 and 7. The xylanase preparations possess activity at pH values from below 5.0 to above 11.0. The xylanase possesses optimum activity at a pH in the range of from pH 5.0 to 9.0. This broad pH range indicates the presence of more than one xylanase compound.

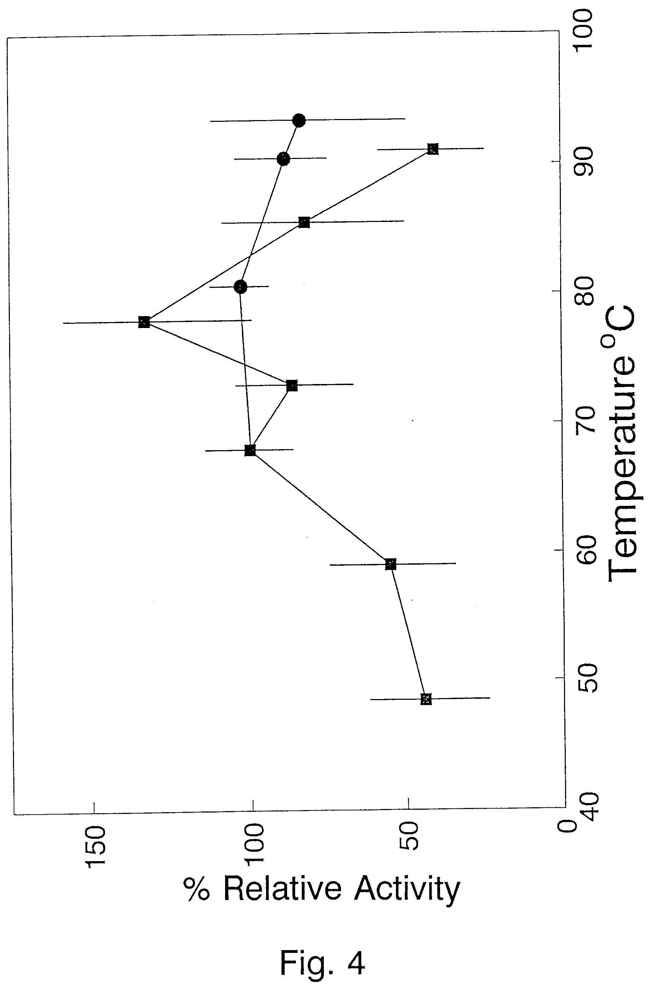

The temperature activity relationship was determined according to Examples 2 and 4, using the method for xylanase activity analysis described in this specification (20 minutes of incubation; pH 6.0). The result of the determination is presented in Figs. 4, 5 and 7. The xylanase possesses activity at temperatures of from below 50°C to above 100°C. The xylanase possesses optimum activity in the range of from 60°C to 90°C, more specifically of from 65°C to 85°C. This broad temperature range also indicates the presence of more than one xylanase compound.

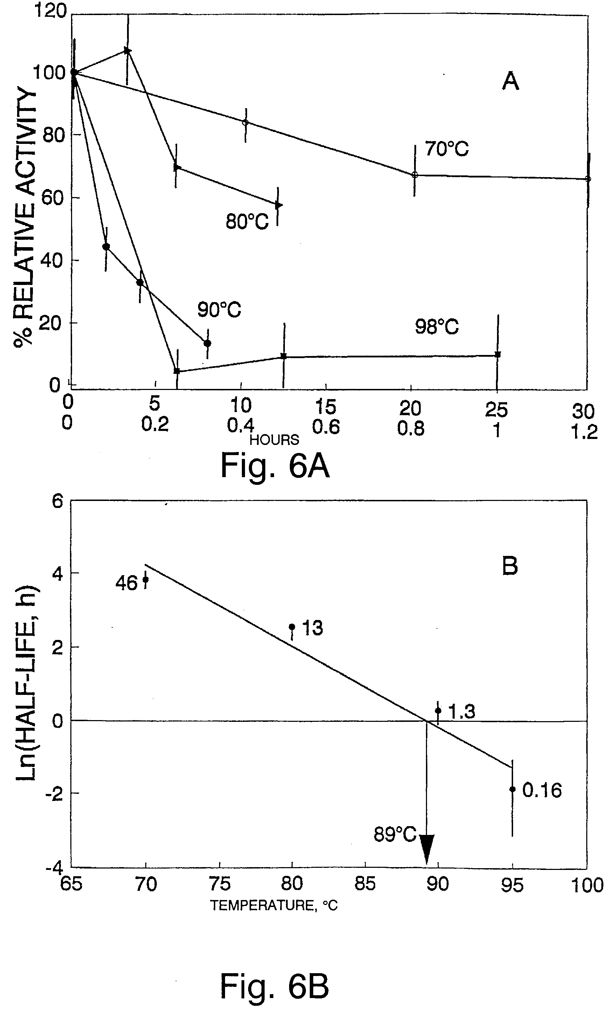

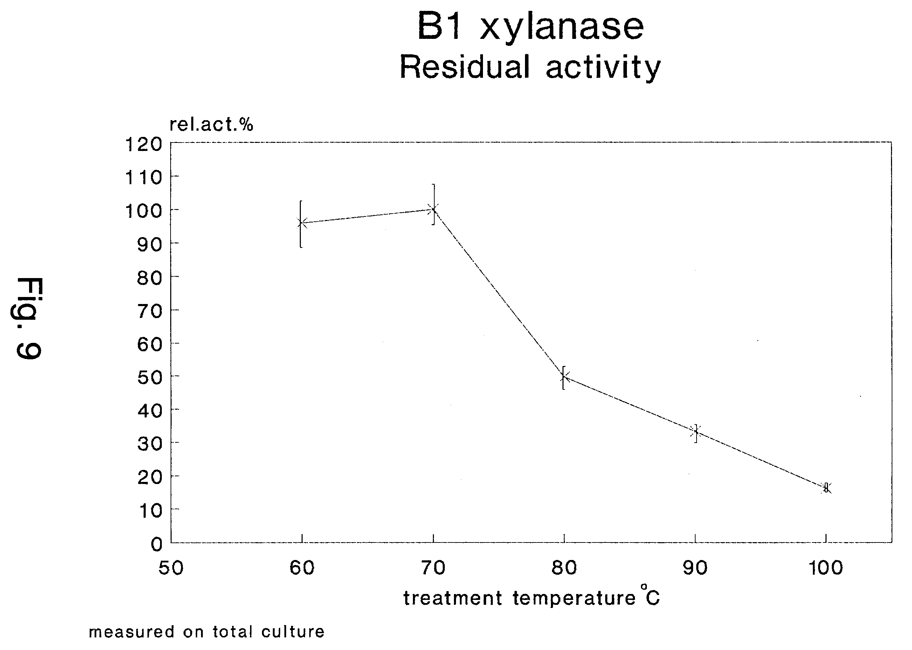

The residual activity of the xylanase was determined according to Examples 2 and 5, using the method for xylanase activity analysis described in this specification (20 minutes of incubation; pH 6.0; 60°C). The result of the determination is presented in Figs. 6A, 6B, and 9. After 10 hours of incubation at 70°C more than 50% residual activity are detectable, preferably more than 70% residual activity. After 10 hours of incubation at 80°C more than 40% residual activity are detectable, preferably more than 50% residual activity. After 4 hours of incubation at 90°C more than 20% residual activity are detectable.

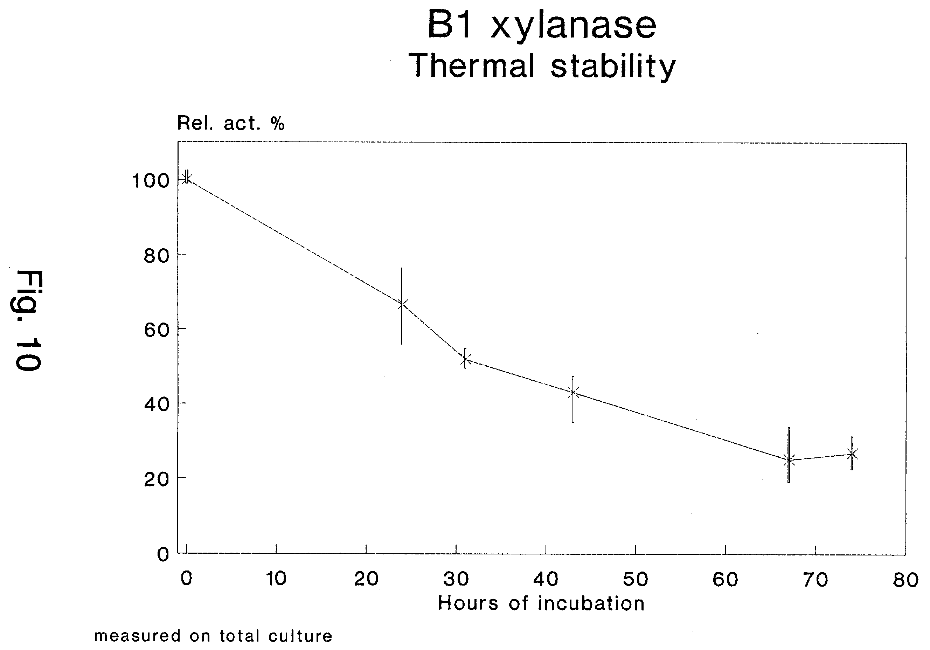

The thermal stability was determined according to Example 2 and 6, using the method for xylanase activity analysis described in this specification (20 minutes of incubation, pH 9.0, 70°C). The result of the determination is presented in Figs. 6B and 10. The half life of the xylanase activity for the crude preparation was found to be between 20 and 40 hours, around 30 hours. Between 20-30% of the xylanase activity remain after 60 hours of incubation.

## Immunochemical Properties

The enzyme preparation of the invention has immunochemical properties identical or partially identical (i.e. at least partially identical) to those of a xylanase derived from Strain B1, DSM No. 6262.

The immunochemical properties can be determined immunologically by cross-reaction identity tests. The identity tests can be performed by the well-known Ouchterlony double immunodiffusion procedure or by tandem crossed immunoelectrophoresis according to *N. H. Axelsen*; Handbook of Immunoprecipitation-in-Gel Techniques; Blackwell Scientific Publications (1983), Chapters 5 and 14.

The terms "antigenic identity" and "partial antigenic identity" are described in the same book, Chapters 5, 19 and 20.

## Xylanase Activity Analysis

Xylanase is determined by assaying for reducing sugars released from oat spelt xylan (XU-method).

The assay is performed with 0.5% oat spelt xylan (Sigma-X-0627), prepared in 40 mM Britton & Robinson buffer (heat treated 30 minutes at 100°C before use), as substrate. The assay is run for 20 minutes at 60°C, using 0.100 ml of enzyme solution and 0.100 ml of substrate, both preheated to 60°C. The mixture is incubated for 20 minutes at 60°C. Then 0.200 ml solution I (35.1 g $Na_2HPO_4,2H_2O$; 40.0g $KNaC_4H_4O_6$, $4H_2O$, suspended in 500 ml deionized $H_2O$ add 110 ml 1N NaOH; 8.0 g $CuSO_4,5H_2o$; 180 g $Na_2SO_4$; add deionized to a total volume of 1 litre) is added. The solution is then heated to 100°C for 20 minutes, and 0.200 ml solution II (50 g $(NH_4)_6Mo_7O_{24},4H_2O$ suspended in 900 ml deionized $H_2O$; 42 ml Concentrated $H_2SO_4$; 6.0 g $Na_2HAsO_4$, $7H_2O$ ad deionized to a total volume of 1 litre) is added. 2.0 ml deionized water are added, and the absorbance on a spectrophotometer (PYE UniCAM PU8600UV/Vls, Phillips) at 520 nm measured.

The reducing sugars are calculated from a standard curve prepared with xylose (40-400 $\mu$g/ml). One XU

is equivalent to 1 nmol xylose released per second per millilitre or per gram of culture broth.

Processes For Treatment Of Lignocellulosic Pulp

In a further aspect, the invention relates to a method for enzymatic treatment of lignocellulosic pulp, comprising employment of an enzyme of this invention.

Enzymatic treatment of lignocellulosic pulp improves the bleachability of the pulp and/or reduces the amount of chemicals necessary for obtaining a satisfactory bleaching.

Due to its temperature stability, the enzyme of the invention may also be applied in a complexing stage of the pulp process, prior to hydrogen peroxide or ozone bleaching.

For use of a xylanase of the invention for delignification of lignocellulosic pulp, the xylanase should preferably be provided in the form of a granulate, preferably a non-dusting granulate, a liquid, in particular a stabilized liquid, a slurry, or a protected enzyme.

In a further preferred embodiment, the agent contains the xylanase in amounts of at least 20%, preferably at least 30%, of the total enzyme protein.

The xylanolytic activity can be measured in xylanase units. In this specification two kinds of units are used: FXU and EXU. By an analytical method a xylanase sample is incubated with remazol-xylan substrate. The background of non-degraded dyed substrate is precipitated by ethanol. The remaining blue colour in the supernatant is proportional to the xylanase activity, and the xylanase units are then determined relatively to an enzyme standard at standard reaction conditions.

The analytical method and the standard reaction conditions are described in two folders: AF 293.6/1 (FXU) and AF 293.9/1 (EXU). FXU is determined at pH 6.0 and EXU is determined at pH 9.0. However, FXU and EXU express enzymatic activity in the same order of magnitude. The folders AF 293.6/1 and 293.9/1 are available upon request to Novo Nordisk A/S, Denmark, which folders are hereby included by reference.

Preferably, the process of the invention is performed at temperatures between 40 and 100°C, more preferred between 50 and 90°C, most preferred between 60 and 80°C.

In another preferred embodiment of the process according to the invention, the enzymatic treatment is performed at a pH above 5.0, more preferred above 6.0, most preferred above 7.0.

In yet another preferred embodiment of the process according to the invention, the enzymatic treatment is performed within a period of 5 minutes to 24 hours, more preferred within 15 minutes to 6 hours, most preferred within 20 minutes to 3 hours.

A suitable xylanase dosage will usually correspond to a xylanase activity of 10 to 5000 FXU/kg or EXU/kg dry pulp, more preferred 100 to 5000 FXU/kg or EXU/kg dry pulp.

In a further preferred embodiment of the process according to the invention, the enzymatic treatment takes place at a consistency of 3-35%, more preferred 5-25%, most preferred 8-15%. The consistency is the dry matter content of the pulp. A pulp with a consistency above 35% is difficult to mix effectively with the enzyme preparation, and a pulp with a consistency below 3% carries too much water, which is a disadvantage from an economic point of view.

In several other preferred embodiments, the xylanases of this invention can be implemented in processes for treatment of lignocellulosic pulp essentially as described in e.g. International Patent Application PCT/DK91/00239, or International Patent Publication WO 91/02839.

The invention is further illustrated in the following examples, which are not intended to be in any way limiting to the scope of the invention as claimed.

**EXAMPLE 1**

Production of Strain B1 Xylanase

A Xylanase preparation of strain B1 can be produced as follows.

Strain B1 (DSM 6262) is grown in a 1 l glass flask for 2 days at 70°C on the medium as described below.

Composition of the strict anaerobic medium for strain B1, DSM 6262:

| NH$_4$Cl | 1.0 g/l |
|---|---|
| NaCl | 0.1 g/l |
| MgCl$_2$ | 0.1 g/l |
| CaCl2 | 0.05 g/l |
| K$_2$HPO$_4$,3H$_2$O | 0.4 g/l |
| Yeast Extract | 0.75 g/l |
| Beech Xylan (Lenzing) | 4.0 g/l |
| Resazurin | 0.0005 g/l |
| Trace metals*) | 1.0 ml |
| Vitamin solution | 1.0 ml |
| NaHCO$_3$ | 3.0 g/l |
| H$_2$O | q.s. to 1 l |

*) Trace metal solution composition

| FeCl$_2$•4H$_2$O | 2.0 g/l | H$_3$BO$_3$ | 0.05 g/l |
|---|---|---|---|
| ZnCL$_2$ | 0.05 g/l | CuCl$_2$ | 0.03 g/l |
| MnCl$_2$ | 0.05 g/l | (NH$_4$)$_6$Mo$_7$O$_{24}$•4H$_2$O | 0.05 g/l |
| AlCl$_3$ | 0.05 g/l | CoCl$_2$•6H$_2$O | 0.05 g/l |
| NiCl$_2$ | 0.05 g/l | EDTA | 0.50 g/l |
| Na$_2$SeO$_3$•5H$_2$O | 0.10 g/l | Conc. HCl | 1.0 ml |

Flush and dispense 10 ml per bottle under N$_2$/CO$_2$ (4:1). Stopper with O$_2$-impermeable rubber stoppers and autoclave at 140°C for 20 min. Add 0.1 ml DSM vitamin solution #141 from filter sterilized anaerobic solution and 0.2 ml of 2.5 g/l Na$_2$S•9H$_2$O from autoclaved stock solution just before inoculation with sterile anaerobic syringe technique. Inoculate with 10% v/v from late exponential phase or stationary phase culture. For some of the following experiments incubation for 2 days at 68°C was applied, for others of the following experiments incubation for 2 days at 78°C was applied.

The strain *D. thermophilum*, DSM 3960, was cultivated in a similar way.

Growth Conditions

The effect of pH and temperature on the growth of Strain B1 in the above medium was investigated. Different pH values in the medium were obtained by varying the concentrations of HCO$_3$ in the medium and the CO$_2$ content of the headspace gas. The buffer was necessarily weak at higher pH values but all growth rate measurements were obtained early in the growth curve and the pH was measured to verify that it had not changed significantly. The cultures were grown in the dark at 68°C.

The turbid, xylan-containing medium interfered with cell measurements by turbidic methods and protein measurement, so specific growth rates, $\mu$ (h$^{-1}$), for the identification of the optimum temperature and pH for growth, were determined from the specific acetate production rate. The correlation of acetate production and cell number was determined by counting cells in a Petroff-Hauser counting chamber after treatment with 0.025% sodium dodecyl sulfate to dissolve the spherical structures and separate the cells from each other (*vide Svetlichnii and Svetlichnaya, supra*).

The effects of pH and temperature on the growth rate of Strain B1 are shown in Figs. 1-2.

Enzyme Preparations

For some of the following studies the total culture was harvested in late exponential phase or early stationary phase and lyophilized, thereby releasing intramolecular and membrane bound enzymes, giving a total of 4 g of powder. This xylanase preparation, which is a total culture, hereafter called P-037, is used as a crude xylanase preparation for further investigation according to Examples 3-8 below.

For other of the following studies the culture supernatant, harvested in late exponential phase or early stationary phase, was clarified by centrifugation at approximately 5000 xg for 20 minutes. This xylanase preparation, which is a culture supernatant containing extracellular soluble enzymes, is used as a crude xylanase preparation for further investigation according to Example 2 below.

**EXAMPLE 2**

Effect of pH and Temperature on the Supernatant Xylanases

The following characterization was performed on the supernatant xylanase preparation from Strain B1, and the strain *D. thermophilum*, DSM 3960, respectively, obtained as described in Example 1. The XU-method described earlier in this specification was used, and all assays are performed for 20 minutes at 60°C.

For pH characterization the assay was run in the interval pH 5-10, and the culture supernatant incubated at 68°C was applied. The result is presented in Fig. 3. Both of the xylanase preparations (Strain B1 and the strain *D. thermophilum*, DSM 3960, respectively) possess xylanolytic activity in a range of from below pH 5 to approximately pH 10. The xylanase preparations are characterized by a broad pH activity range, showing pH optimum in the range of from pH 5.0 to pH 9.5, more specifically pH 5.5 to pH 9.0. This broad activity range indicates the presence of more than one xylanase component in the preparation.

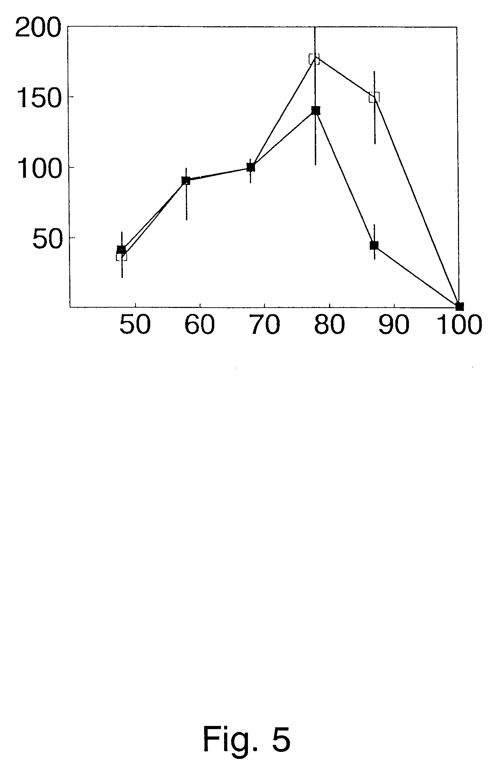

For temperature characterization the assay was run in the interval of from approximately 50°C to approximately 90°C. In one experiment the culture supernatant incubated at 68°C was applied. Both of the xylanase preparations (Strain B1 and the strain *D. thermophilum*, DSM 3960, respectively) possess xylanolytic activity in a range of from below 48°C to above approximately 90°C. Both xylanase preparations show maximal activity near 80°C, Strain B1 shows two local temperature optima, one near 70°C and the other near 80°C. The activity at 80°C was 30 to 50% higher than the activity at 70°C. The soluble enzymes retained 30 to 80% activity at 90°C compared to 80°C. The results are presented in Figs. 4 and 5.

In a second experiment, the culture supernatant incubated at 78°C was applied for temperature characterization, and the temperature profile of the soluble xylanases was significantly different from the profile described above. Xylanase preparations obtained from cultures incubated at 78°C were more active at high temperatures, having a substantial amount of activity at temperatures above approximately 92°C. The results are presented in Figs. 4 and 5.

Thermostability of the Supernatant Xylanase

The percent activity remaining, relative to untreated samples, of the culture supernatant of Strain B1 incubated at 68°C, without substrate present, is presented in Figs. 6A and 6B.

As appears from the Figures, the xylanases of the invention are very thermostable. After 10 hours of incubation at 70°C more than 50% residual activity are detectable, preferably more than 70% residual activity. After 10 hours of incubation at 80°C more than 40% residual activity are detectable, preferably more than 50% residual activity. After 4 hours of incubation at 90°C more than 20% residual activity are detectable.

Fig. 6B shows a semi-logarithmic plot of the thermal half-life of the activity of the supernatant xylose preparation of the invention as a function of temperature. At 70°C the thermal half-life was more than 40 hours, approximately 46 hours. At 80°C the thermal half-life was more than 10 hours, approximately 13 hours. At 90°C a substantial amount of activity is still present, approximately 1 hour.

The data form a straight line ($r^2 > 95\%$), and the thermal activity number, i.e. the temperature leading to a half-life of 1 hour, was 89°C.

**EXAMPLE 3**

pH-optimum

pH-characterization of the xylanase activity in Strain B1 is determined using the crude xylanase preparation P-037 described in Example 1, and the XU-method described earlier in this specification, performed in the pH range of 5 to 11. All assays are performed for 20 minutes at 60°C. The pH optimum of the crude preparation is between pH 5 and 7 (Fig. 7). Between 10-20% of the xylanase activity persist at pH 9. Taking the observations from Example 2 into account, and the fact that activity of the enzyme misture is presented as % relative activity, pH profile of Fig. 7 may be due to the presence of a substantially larger amount of one or more different xylanolytic enzymes having pH optimum around 6.

**EXAMPLE 4**

Temperature Optimum

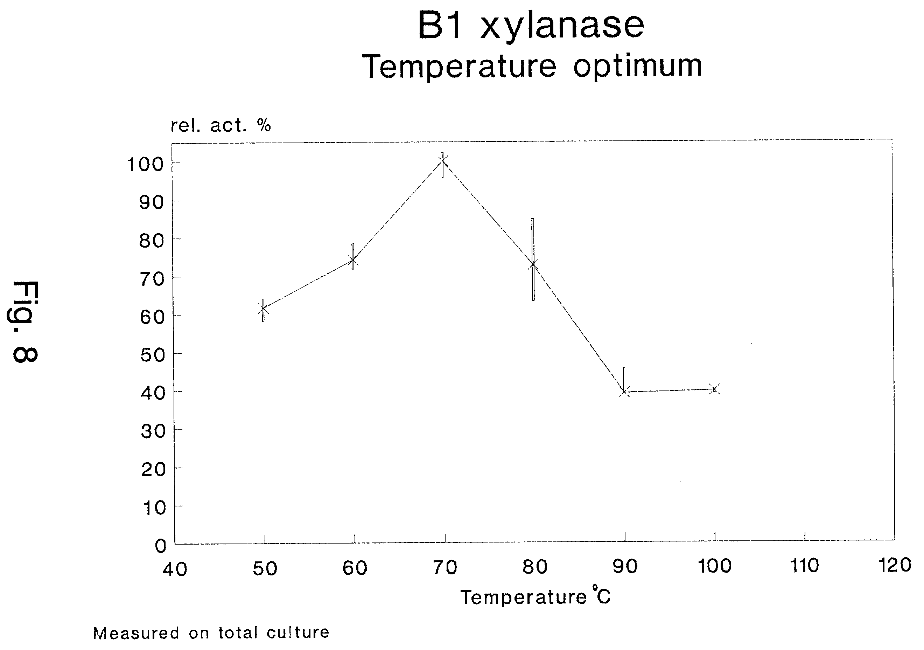

The temperature profile of the xylanase activity in B1 is determined by using the crude xylanase preparation P-037, described in Example 1 and the XU-method described earlier, performed at pH 6 in the temperature range of 50-100°C. All assays are performed for 20 minutes. The temperature optimum of the crude preparation is found to be between 60 and 80°C (Fig. 8), indicating an optimum around 70°C. Between 30-40% of the xylanase activity remain at 90°C.

**EXAMPLE 5**

Residual Activity

The thermal stability of the xylanase of B1 is measured as residual activity after heat treatment using the crude xylanase preparation P-037, described in Example 1. Xylanase solutions are heated at 60 to 100°C for half an hour and hereafter the residual xylanase activity is measured using the XU-method described earlier, at pH 6, 60°C for 20 minutes. The result of the determination is presented in Fig. 9. From this Figure it appears that the xylanase possesses a residual activity after 20 minutes at 70°C of more than 50%, more specifically more than 70%, most specifically more than 90%. After 20 minutes at 80°C the xylanase possesses a residual activity of more than 20%, more specifically more than 40%. After 20 minutes at 90°C the xylanase possesses a residual activity of more than 20%.

**EXAMPLE 6**

Thermal Stability

Thermal stability of the xylanase activity in B1 was determined by using the crude xylanase preparation P-037, described in Example 1 and the XU-method described earlier. Solutions of the P-037 were incubated at 70°C, pH 9. Samples were taken with appropriate intervals, and the residual activity was measured using the XU-method described earlier, at pH 9, 70°C for 20 minutes. The result of the determination is presented in Fig. 10. The half-life of the xylanase activity for the crude preparation was found to be between 20 and 40 hours, around 30 hours. Between 20-30% of the xylanase activity remain after 60 hours of incubation.

**EXAMPLE 7**

pI of the Xylanase Activity

In a first experiment, the pI of the xylanase activity was determined using LKB ampholine PAG plates pH 3.5-9.5 and a solution of the crude xylanase preparation P-037, described in Example 1. After the electrophoresis the gel is washed twice for 15 minutes, once in water, once in trisbuffer pH 9, and then overlayered with a thin coat of detection agar consisting of 0.5% of oat spelt xylan, 1% of agarose pH 9. The overlayered gel is incubated overnight at 50°C. The xylanase activity was visualized using Congo Red staining (staining for 10 minutes with 0.1% of Congo Red and destained for 2 X 15 minutes in 1 M NaCl). Congo Red could be detected with pI values between 4 and 7.4 components with xylanolytic activity could be detected in the range of from 4 to 7.

In a second experiment, the pI of the xylanase activity was determined also using LKB ampholine PAG plates pH 3.5-9.5 and a solution of the crude xylanase preparation P-037, described in Example 1. Then, in a first set of tests, the solution was subjected to electrophoresis without any intermediate treatment, and in a second set of tests, the solution was heat treated at 70°C at pH 10 (0.1 M bicarbonate buffer) for 60 minutes.

After the electrophoresis, the gels were washed twice for 20 minutes in 0.5 M Trisbuffer, pH 7.0, and in one set of tests overlayered with a thin coat of detection agar consisting of 0.5% of oat spelt xylan, 1% of agarose, pH 6.0 (with 0.1 M phosfate buffer), and in a second set of tests overlayered with a thin coat of detection agar consisting of 0.5% of oat spelt xylan, 1% of agarose, pH 10.0 (with 0.1 M bicarbonate buffer).

The gels were incubated at 70°C overnight and washed for 20 minutes in 0.5 M Trisbuffer, pH 7.0. The xylanase activity was visualized using Congo Red staining (staining for 10 minutes with 0.1% of Congo Red and destained for 2 X 15 minutes in 1 M NaCl).

In parallel an isoelectric focusing standard was run on a mixture of proteins with known pI, and after commassie-staining the pI of the xylanase activity could be identified.

The results of this experiment are presented in Table 2, below.

Table 2

| pI of Xylanase Activity | | |
|---|---|---|
| pH in overlayered gel | Heat treated (70°C/pH10/60min) | No heat treatment |
| pH 6.0<br>pH 10.0 | 4.9; (8.2); 8.9<br>5.8; 6.8; 8.9 | 4.9; 5.9; 7.4; (8.2); 8.9<br>4.7; 8.9 |

Apparently, the preparation contains a complex of enzymes with xylanolytic activity. Besides, the pI determined above (4 components with xylanolytic activity in the range of from 4 to 7) additional pI values for xylanase activity were determined at 7.4, 8.2, and 8.9, among which the band at 8.9 was the most powerful. Activity blur was observed between 7.4 and 8.9, which is the reason for the figure in the bracket (8.2).

The xylanase activity at 8.9 was observed at any of the tests.

**EXAMPLE 8**

Bleach Boosting

In this example the bleach boosting effect is demonstrated by a screening method. Being a screening method the experiment, therefore, is not conducted at industrially relevant conditions.

For this experiment 2 portions of 5 g of dry pulp, Swedish $O_2$ delignified pine Kraft, were soaked in water for 8 hours and afterwards re-pulped for 3 minutes in Britton-Robinson buffer pH 6.0. Excess liquid was gently squeezed out of the pulp, whereafter the pulp was transferred to 250 ml Erlenmeyer Flasks and suspended in fresh buffer to a consistency of 3.5% dry substance. The two pulp portions were preincubated at 70°C for 15 minutes whereafter the enzyme was added to the one flask at a dosage of around 100 EXU/kg dry pulp. The control flask received water. The hydrolysis took place at 70°C (pH 6) for 48 hours, whereafter the liquid was drained off on a Buchner funnel. From the filtrate was taken out samples for absorbance measurements (released lignin gives absorbance at 280 nm) and for determination of the type of attacked carbohydrate (hemicellulose and/or cellulose), *vide infra*. The pulp was washed and plates were made for determining the Kappa Number. The Kappa Number is a measure of the lignin content and is defined in SCAN-C **1** 77 (Scandinavian Pulp, Paper and Board Testing Committee).

A sample of the filtrate after the enzyme treatment step was filtered through a 0.45 $\mu$m filter, freeze dried, hydrolyzed for two hours with 2 N-trifluoroacetic acid (TFA) at 100°C, whereafter the TFA was removed by evaporation and the sample redissolved in demineralized water. The monosaccharide composition of the hydrolyzed sample was then determined by means of an Ion Exchange HPLC system followed by electrochemical detection (for reference see eg. *Hardy et al.* (1988); Analytical Biochemistry, **170** 54-62).

The Kappa Numbers were analyzed to 13.5 for the control and 12.7 for the enzyme treated pulp. This shows a reduction of 6%, which is a significant improvement at this step in the process.

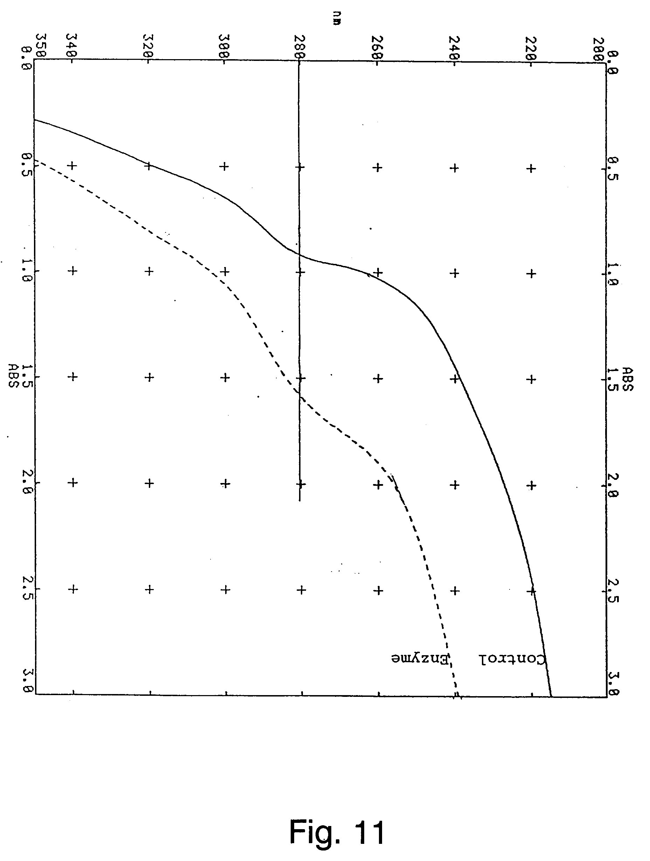

The absorbance scan curves are shown in Fig. 11. Lignin is known to absorb light around 280 nm and the curves clearly demonstrate that more lignin is released from the enzyme treated pulp than from the control.

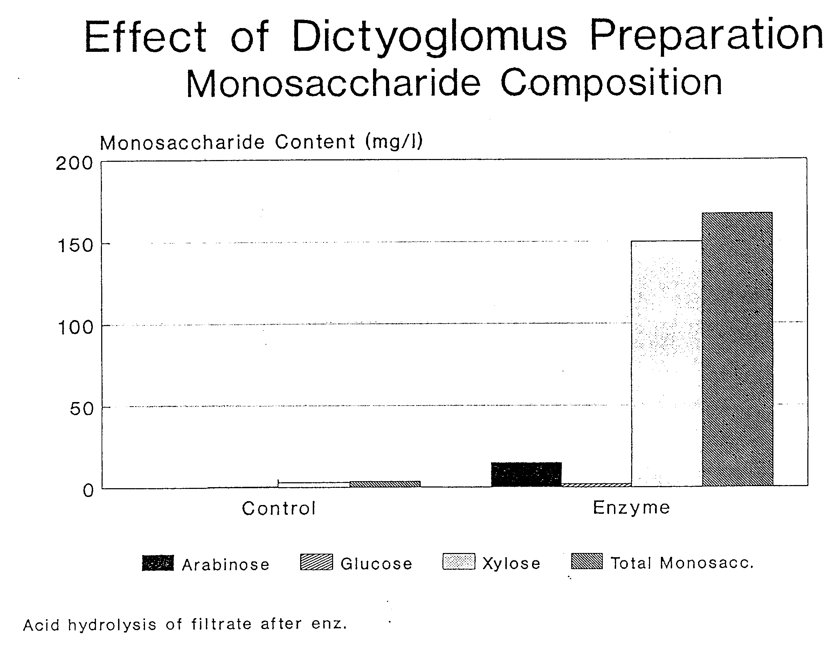

The monosaccharide composition of the acid hydrolyzed filtrates is shown in Fig. 12 below. First, it is seen that there is a significant effect of the enzyme compared to the control, and second, that the xylose comprises 90% of the total monosaccharides whereas glucose is hardly present (-1%). This clearly demonstrates xylanase action with very little cellulase present. This is positive as cellulase action can give rise to yield or strength losses in the final paper.

The three above mentioned methods all demonstrate that the enzymes of this invention have a potential bleach boosting effect on paper pulp.

**Claims**

1. A biologically pure culture of a species belonging to the genus *Dictyoglomus*, which has the ability to produce xylanases.

2. The culture according to claim 1, which microorganisms have morphological, physiological and growth properties essentially as described in the specification.

3. A biologically pure culture of a strain of microorganisms being essentially identical with the strain DSM No. 6262.

4. An enzyme preparation having xylanase activity, and having the following characteristics:
   (a) pH optimum in the range of from pH 5.0 to pH 9.0 (after 20 minutes at 60°C);
   (b) temperature optimum in the range of from 60 to 90°C (after 20 minutes at pH 6.0); and
   (c) immunochemical properties identical or partially identical to those of a xylanase derived from Strain B1, DSM No. 6262.

5. An enzyme preparation according to claim 4, being obtainable from a strain of the genus *Dictyoglomus*.

6. An enzyme preparation according to claim 5, being obtainable from a strain of *D. thermophilum* or *D. turgidus*.

7. An enzyme preparation according to claim 6, being obtainable from Strain B1, DSM No. 6262, or the strain *D. thermophilum*, DSM 3960, or mutants or variants thereof.

8. An enzyme preparation according to any of claims 4-7, further **characterized** by having:
   (a) a relative residual activity after 20 minutes at 70°C and pH 6.0 of more than 50%, preferably more than 70%; and
   (b) a half-life of more than 15 hours, more preferred more than 20 hours, most preferred more than 25 hours, determined at 70°C and pH 6.0.

9. A process for the manufacture of a xylanase preparation according to any of claims 4-8, which process comprises cultivation of a xylanase producing strain of an organism according to any of claims 1-3 in a suitable nutrient medium, containing carbon and nitrogen sources and inorganic salts, followed by recovery of the desired enzyme by methods known *per se*.

10. The process according to claim 9, in which a strain of an microorganism essentially identical to the strain DSM No. 6262, or the strain *D. thermophilum*, DSM 3960, or mutants or variants thereof, is cultivated.

11. A process for treatment of lignocellulosic pulp, in which the lignocellulosic pulp is treated with an enzyme preparation according to any of claims 4-8.

12. The process according to claim 11, which process is performed at temperatures between 40 and 100°C, preferably between 50 and 90°C, most preferred between 60 and 80°C.

13. The process according to either of claims 11-12, which process is performed at a pH above 5.0, more preferred above 6.0, most preferred above 7.0.

14. The process according to any of claims 11-13, which process is performed within a period of 5 minutes to 24 hours, preferably within 15 minutes to 6 hours, most preferred within 20 minutes to 3 hours.

15. The process according to any of claims 11-14, in which process the enzyme dosage corresponds to xylanase activity of 10 to 5000 FXU/kg or EXU/kg dry pulp, more preferred 100 to 5000 FXU/kg or EXU/kg dry pulp.

16. The process according to any of claims 11-15, which process is performed at a consistency of 3-35%, more preferred 5-25%, most preferred 8-15% dry substance.

**17.** An agent containing a xylanase preparation according to any of claims 4-8, provided in the form of a granulate, preferably a non-dusting granulate, a liquid, in particular a stabilized liquid, a slurry, or a protected enzyme.

**18.** An agent according to claim 17, in which the xylanase preparation constitutes at least 20%, preferably at least 30%, of the total enzyme protein.

EFFECT OF pH ON STRAIN B1

SPECIFIC GROWTH RATE, $\mu$ (h$^{-1}$)

Fig. 1

EFFECT OF TEMPERATURE ON STRAIN B1

Fig. 2

Fig. 3

Fig. 4

Fig. 5

Fig. 6A

Fig. 6B

B1 xylanase
pH-optimum

rel. act.%

pH

Measured on total culture

Fig. 7

21

# B1 xylanase
## Temperature optimum

rel. act. %

Temperature °C

Measured on total culture

Fig. 8

EP 0 511 933 A2

B1 xylanase
Residual activity

Fig. 9

# B1 xylanase
## Thermal stability

Rel. act. %

Hours of incubation

measured on total culture

Fig. 10

EP 0 511 933 A2

Fig. 11

# Effect of Dictyoglomus Preparation
## Monosaccharide Composition

Monosaccharide Content (mg/l)

Acid hydrolysis of filtrate after enz.

EP 0 511 933 A2